# EUROPEAN PATENT SPECIFICATION

(11) **EP 1 208 181 B1**
(45) Date of publication and mention of the grant of the patent: **06.10.2004**
(21) Application number: 00964030.1
(22) Date of filing: 21.08.2000
(51) Int. Cl.: C09J 7/02, C09J 123/08, A61L 15/58, B32B 27/32

(54) **HOT MELT PRESSURE SENSITIVE ADHESIVES FOR DIAPER CONSTRUCTION AND POSITIONING**
SELBSTKLEBENDER HEIZSCHMELZKLEBER FÜR WINDELKONSTRUKTION UND POSITIONIERUNG
ADHESIFS THERMOFUSIBLES AUTOCOLLANTS UTILISES DANS LA FABRICATION ET LE POSITIONNEMENT DES COUCHES

(30) Priority: 24.08.1999 US 150411 P
(43) Date of publication of application: 29.05.2002
(73) Proprietor: KRATON Polymers Research B.V., 1031 CM Amsterdam (NL)
(72) Inventor: DE KEYZER, Noel, Raymond, Maurice, B-1348 Ottignies, Louvain-la-Neuve (BE); STONER, Carolyn, Ann, Houston, TX 77053 (US)
(74) Representative: Kortekaas, Marcellinus C. J. A.
(86) International application number: PCT/EP2000/008187
(87) International publication number: WO 2001/014487

(56) References cited:
- WO-A-89/01002
- WO-A-98/15407
- WO-A-99/37730

## Description

### Field of the Invention

This invention relates to hot melt pressure sensitive adhesives typically for use with absorbent articles. The hot melt pressure sensitive adhesive is especially useful as diaper construction adhesive or positioning adhesive.

### Background of the Invention

Positioning adhesives are used on disposable articles (absorbent articles) such as sanitary napkins, incontinent pads, bed pads, feminine pads, panty shields, diaper inserts, etc. where an adhesive layer is used to attach the article to a woven fabric substrate such as a supporting undergarment or bed sheet. The positioning adhesive is commonly applied to a release liner and transfer coated to the garment facing surface of the disposable article. The positioning adhesive must be capable of attaching to the undergarment to hold the article in place without transferring to or otherwise being deposited on the undergarment. Furthermore, the adhesive must not discolour, damage, or disturb the fibres of the garment.

The positioning adhesive must be a pressure sensitive adhesive that has an application viscosity that permits it to readily flow onto and partially penetrate the particular surface to which it is applied. It must have good bond strength and high tack for initial placement of the article on the undergarment but also must have the ability to avoid loss of adhesion over time.

These articles are sometimes used for long periods of times at body temperature and they can have the drawback that the hot melt adhesive gradually softens and penetrates into the undergarment to which the article is adhering. In this case, the adhesive force greatly increases and the cohesive force is reduced. This causes the adhesive layer to suffer cohesion breakdown when the article is removed and some adhesive remains on the undergarment. Prevention of this deposit of adhesive on the undergarment is accordingly a necessary prerequisite for a successful positionable hot melt adhesive composition.

Block copolymers of styrene and dienes such as butadiene or isoprene have been used for a number of years in positionable hot melt adhesive formulations. More recently, the material of choice for such adhesives in feminine care applications has been hydrogenated block copolymers of styrene and butadiene such as KRATON G1650 SEBS (hydrogenated styrene-butadiene-styrene) block copolymer (KRATON is a trademark). Formulations based on these SEBS block copolymers have been found to have excellent adhesion to fabrics like cotton and nylon and have the advantage that they leave no residue after peeling. The application viscosity of formulations using these polymers is acceptable but it would be advantageous to have a positionable adhesive formulation which has a lower viscosity in order to lower the application temperature. This reduces the risk of degradation, char forming, and filter plugging. This also results in energy and cost savings, decreases maintenance costs, and reduces the amount of odour due to any volatiles coming from the adhesive. The present invention provides such an improved hot melt adhesive formulation.

### Summary of the Invention

The present invention relates to a hot melt pressure sensitive adhesive which comprises:
(a) from 5 to less than 30 percent by weight basis the total of (a), (b) and (c) of a homogeneous linear or substantially linear interpolymer of ethylene and at least one C₃-C₂₀ alpha olefin having a density of at least 0.85 grams per cubic centimetre and a melt flow index below 30 dg/min; and
(b) from 50 to 80 percent by weight basis the total of (a), (b) and (c) of a tackifying resin; and
(c) from 5 to 35 percent by weight basis the total of (a), (b) and (c) of a plasticizer.

Although throughout this specification interpolymer, tackifying resin and plasticizer are used in their singular form, it will be appreciated that blends of two or more interpolymers, two or more tackifying resins and/or two or more plasticizers are also within the scope of the invention as defined herein.

The present invention further relates to an absorbent article which incorporates an adhesive.

### Detailed Description of the Invention

As discussed above, the adhesive of the present invention contains from 5 to less than 30 percent by weight of a homogeneous linear or substantially linear interpolymer of ethylene and a C₃-C₂₀ alpha olefin. At least 5 percent is necessary to get the desired pressure sensitive adhesive properties and for the adhesive to be sufficiently cohesive. It is preferred that the maximum amount of the blend be less than 30 percent by weight in order to keep the viscosity of the adhesive sufficiently low for the adhesive application. For the same reason, it is preferred that from 10 to 15% by weight of the interpolymer be used especially if the adhesive is to be used as positioning adhesive. It the adhesive is intended to be used as construction adhesive for disposable diapers then the amount of interpolymer most preferably ranges from 20 to less than 30% by weight of the composition. More polymer than 30 percent can be used and good adhesion properties will be obtained but the viscosity will be unnecessarily increased.

The homogeneous linear or substantially linear interpolymers of ethylene and a C₃-C₂₀ alpha olefin preferably have a density from 0.850 to 0.910 g/cm³, a melt flow index (MFI) lower than 30, preferably less than 15, dg/min (190C/2.16kg), and a polydispersity of less than 2.2. The interpolymers may have densities higher than the preferred range as long as the resulting formulation has a viscosity and adhesive properties within the preferred range.

The term interpolymer is used herein to indicate a copolymer, or a terpolymer, or the like. That is, at least one other comonomer is polymerized with ethylene to make the interpolymer. The homogeneous linear or substantially linear polymer can an ethylene polymer prepared using a constrained geometry or single site metallocene catalyst. By the term homogeneous, it is meant that any comonomer is randomly distributed within a given interpolymer molecule and substantially all of the interpolymer molecules have the same ethylene/comonomer ratio within that interpolymer. The melting peak of homogeneous linear and substantially linear ethylene polymers, as determined by differential scanning calorimetry (DSC), will broaden as the density decreases and/or as the number average molecular weight decreases. However, unlike heterogeneous polymers, when a homogeneous polymer has a melting peak greater than 115 °C (such as is the case of polymers having a density greater than 0.940 g/cm³) such polymers typically do not additionally have a distinct lower temperature melting peak. The homogeneous linear or substantial linear ethylene polymers are characterized as having a narrow molecular weight distribution (Mw/Mₙ). For the linear and substantially linear ethylene polymers, the M_{w}/Mₙ is preferably from 1.5 to 2.5, more preferably from 1.8 to 2.2.

Substantially linear ethylene polymers are homogeneous polymers having long chain branching. The long chain branches have the same comonomer distribution as the polymer backbone and can be as long as about the same length as the length of the polymer backbone. When a substantially linear ethylene polymer is employed in the practice of the invention, such polymer will be characterized as having a polymer backbone substituted with from 0.1 to 3 long chain branches per 1000 carbons. Methods for determining the amount of long chain branching present, both qualitatively and quantitatively, are known in the art. For qualitative and quantitative methods for determination, see US Patent Nos. 5,272,236 and 5,278,272 which are herein incorporated by reference.

The homogeneous linear or substantially linear ethylene polymer will be an interpolymer of ethylene with at least one alpha olefin. Preferred are interpolymers of ethylene with at least one C₃-C₂₀ alpha olefin (for instance, propylene, isobutylene, 1-butene, 1-pentene, 1-hexene, 4-methyl-1-pentene, and 1-octene) with interpolymers of ethylene with at least one C₄-C₂₀ alpha olefin, particularly at least one C₆-C₈ alpha olefin, being most preferred. When 1-octene is employed as the comonomer, preferably the 1-octene is present in an amount greater than 14 percent by weight in the polymer as measured by NMR in accordance with ASTM D-5017. More preferably, the 1-octene comonomer content is greater than 20 percent by weight.

Homogeneously branched linear ethylene/alpha olefin interpolymers may be prepared by using polymerization processes which provide a homogeneous short chain branching distribution. For instance, see U.S. Patent 3,645,992 which is herein incorporated by reference. In this process, a soluble vanadium catalyst system is used. Others have used so-called single site metallocene catalyst systems to make such polymers. Substantially linear ethylene/alpha olefin interpolymers are available from the Dow Chemical Company and may be prepared in accordance with the techniques described in U.S. Patents 5,272,236 and 5,278,272 which are herein incorporated by reference. For example, commercial grades are available from DuPont Dow Elastomers under the trade name ENGAGE and from Exxon under the trade name EXACT. In this specification whenever a product is identified by its trademark, the trademark is in capital letters.

As used herein, the term "tackifier" means any of several hydrocarbon based compositions useful to impart tack to the hot melt adhesive composition. For instance, several classes of tackifiers include aliphatic C₅ resins, polyterpene resins, hydrogenated resins, mixed aliphatic-aromatic resins, rosin esters, and hydrogenated rosin esters.

Exemplary tackifying resins useful herein include aliphatic, cycloaliphatic and aromatic hydrocarbons and modified hydrocarbons and hydrogenated versions; terpenes and modified terpenes and hydrogenated versions; and rosins and rosin derivatives and hydrogenated versions; and mixtures thereof. These tackifying resins have a ring and ball softening point from 70 °C to 150 °C, and will typically have a viscosity at 350 °F (177 °C), as measured using a Brookfield viscometer, of no more than 2000 centipoise (20 grams/cm second). They are also available with differing levels of hydrogenation, or saturation, which is another commonly used term. Useful examples include EASTOTAC H-100, H-115 and H-130 from Eastman Chemical Co. in Kingsport, Tennessee, which are partially hydrogenated cycloaliphatic petroleum hydrocarbon resins with softening points of 100 °C, 115 °C and 130 °C, respectively. These are available in the E grade, the R grade, the L grade and the W grade, indicating differing levels of hydrogenation with E being the least hydrogenated and W being the most hydrogenated. The E grade has a bromine number of 15, the R grade a bromine number of 5, the L grade a bromine number of 3 and the W grade has a bromine number of 1. EASTOTAC H-142R from Eastman Chemical Co. has a softening point of about 140 °C. Other useful tackifying resins include ESCOREZ 5300 and 5400, partially hydrogenated cycloaliphatic petroleum hydrocarbon resins, and ESCOREZ 5300 and 5400, partially hydrogenated cycloaliphatic petroleum hydrocarbon resins, and ESCOREZ 5600, a partially hydrogenated aromatic modified petroleum hydrocarbon resin all available from Exxon Chemical Co. in Houston, TX; WINGTACK Extra which is an aliphatic, aromatic petroleum hydrocarbon resin available from Goodyear Chemical Co. in Akron, OH; HERCOLITE 2100, a partially hydrogenated cycloaliphatic petroleum hydrocarbon resin available from Hercules, Inc. in Wilmington, DE; and ZONATAC 105 and 501 Lite, which are styrenated terpene resins made from d-limonene and available from Arizona Chemical Co. in Panama City, FL.

There are numerous types of rosins and modified rosins available with differing levels of hydrogenation including gum rosins, wood rosins, tall-oil rosins, distilled rosins, dimerized rosins and polymerized rosins. Some specific modified rosins include glycerol and pentaerythritol esters of wood rosins and tall-oil rosins. Commercially available grades include, but are not limited to, SYLVATAC 1103, a pentaerythritol rosin ester available from Arizona Chemical Co., UNITAC R-100 Lite, a pentaerythritol rosin ester from Union Camp in Wayne, NJ, PERMALYN 305, a erythritol modified wood rosin available from Hercules and FORAL 105 which is a highly hydrogenated pentaerythritol rosin ester also available from Hercules. SYLVATAC R-85 and 295 are 85 °C and 95 °C melt point rosin acids available from Arizona Chemical Co. and FORAL AX is a 70 °C melt point hydrogenated rosin acid available from Hercules, Inc. NIREZ V-2040 is a phenolic modified terpene resin available from Arizona Chemical Co.

Another exemplary tackifier, PICCOTAC 115, has a viscosity at 350 °F (177 °C) of about 1600 centipoise (16 grams/cm·second)). Other typical tackifiers have viscosities at 350 °F (177 °C) of much less than 1600 centipoise (16 grams/(cm·second)), for instance, from 50 to 300 centipoise (0.5 to 3 grams/(cm·second)).

Exemplary aliphatic resins include those available under the trade designations ESCOREZ, PICCOTAC , HERCURES, WINGTACK, HI-REZ, QUINTONE, TACKIROL, etc. Exemplary polyterpene resins include those available under the trade designations NIREZ, PICCOLYTE, WINGTACK, ZONAREZ, etc. Exemplary hydrogenated resins include those available under the trade designations ESCOREZ, ARKON, CLEARON, etc. Exemplary mixed aliphatic-aromatic resins include those available under the trade designations ESCOREZ, REGALITE, HERCURES, AR, IMPREZ, NORSOLENE-M, MARUKAREZ, ARKON-M, QUINTONE, etc. Other tackifiers may be employed, provided they are compatible with the homogeneous linear or substantially linear ethylene/olefin interpolymer and the oil.

MMAP cloud point is a well-known measure of aromatic solubility and determines the aliphatic/aromatic character of the resin. The lower the MMAP cloud point value, which is expressed in degrees centigrade, the more aromatic is the resin. A 1:2 mixture of methylcyclohexane and aniline is used as the solvent system in the MMAP cloud point determination. A standard weight of resin is dissolved in the solvent at high temperature and allowed to cool with mixing. The temperature at which the resin begins to separate out as an extra phase is determined to be the MMAP cloud point. This may be seen in the mixture as a cloudiness in the previously clear solution.

Preferably, the MMAP cloud point is at least 70 °C, in particular if the adhesive comprises from 20 to less than 30 percent by weight of interpolymer (basis the total of components (a), (b) and (c) as outlined herein before).

Suitable plasticizers include plasticizing oils like low aromatic content (carbon aromatic distribution ≤ 5%, preferably ≤ 2%, more preferably 0% as determined according to DIN 51378) hydrocarbon oils that are paraffinic or naphthenic in character. Those products are commercially available from Shell Oil Company, like SHELLFLEX, CATENEX, ONDINA oils, KAYDOL oil from Witco, or TUFFLO oils from Arco. Other plasticizers include compatible liquid tackifying resins like REGALREZ R-1018. Other ingredients might also be added, like olefin oligomers, low molecular weight polymers (≤ 30,000 g/mol) like liquid polybutene or liquid polyisoprene copolymers, like liquid styrene/isoprene copolymers or hydrogenated styrene/isoprene copolymers and liquid alpha-olefin polymers, vegetable oils and their derivatives, paraffin and microcrystalline waxes. The plasticizer is used in an amount of 5 to 35% by weight. The preferred range is 20 to 35% by weight because below 20% the hot-melt viscosity might be too high and therefore cause processing and application problems. Above 35%, the product is too soft and plasticizer (oil) bleeding can occur. Indeed, plasticizer bleeding through the protective siliconized liner negatively affects the appearance of the product, i.e., it is not attractive from a marketing point of view.

It is known in the art that various other components can be added to modify the tack, the odour, the colour of the adhesives. Antioxidants and other stabilizing ingredients can also be added to protect the adhesive from degradation induced by heat, light and processing or during its shelf life, like during storage. Several types of antioxidants can be used, either primary antioxidants like hindered phenols or secondary antioxidants like phosphite derivatives or blends thereof. Examples of commercially available antioxidants are IRGANOX 565 from Ciba-Geigy (2.4-bis-(n-octylthio)-6-(4-hydroxy-3,5-di-tertiary-butyl anilino)-1,3,5-triazine), IRGANOX 1010 from Ciba-Geigy (tetrakis-ethylene-(3,5-di-tertiary-butyl-4-hydroxy-hydrocinnamate)methane) and POLYGUARD HR from Uniroyal (tris-(2,4-di-tertiary-butyl-phenyl)phosphite).

The positioning adhesive composition of the present invention should have a viscosity of 100 to 10,000 cPs at 177 °C, preferably from 600 to 6,000 cPs at 177 °C. The adhesive must exhibit no transfer after being peeled away from a fabric. The adhesive composition is used in articles such as disposable diapers, sanitary napkins, bed pads, incontinent pads, surgical drapes, plasters and bandages.

### EXAMPLES

Several different types of metallocene interpolymers were tested, an ethylene-butene-1 copolymer (sold under the trademark EXACT) and several ethylene/octene-1 copolymers (sold under the trademark ENGAGE). The table below shows the properties of these polymers. Mₙ is the number average molecular weight, M_{w} is the weight average molecular weight, M_{w}/Mₙ is a measure of the polydispersity, and MFI is the melt flow index expressed in dg/min at 190 °C/2.16 kg. PICCOTAC 95 is a trademark for an aliphatic hydrocarbon tackifying resin which is manufactured by Hercules. TUFFLO 6056 oil is a plasticizing oil which is manufactured by ARCO.

| Grade | density g/cm³ | MFI dg/min | Mₙ | M_{w} | M_{w}/Mₙ |
|---|---|---|---|---|---|
| ENGAGE 8200 | 0.87 | 5 | 65,696 | 122,262 | 1.861 |
| ENGAGE 8400 | 0.87 | 30 | 39,930 | 82,881 | 2.076 |
| | | | | | |
| EXACT 4049 | 0.873 | 4.5 | 57,005 | 122,743 | 2.153 |

All the ingredients were compounded in a Z-blade mixer. Then the samples were put in beaker in an oven at 180 °C. Once molten, the adhesive was poured onto a Mylar sheet and cast to obtain a thickness of 2 mils (50 µm). Prior to testing, the samples were conditioned at 23 °C -50 %RH (relative humidity) for 24 hours.

Standard peel, tack and cohesion tests were carried out on these formulations. To assess the right functionality of the adhesive, specific adhesion tests on fabrics were performed, namely to evaluate the adhesion of the positioning adhesive onto the undergarment. Cotton and nylon fabrics are the two reference materials used in these tests.

The following peel adhesion tests on fabric were carried out:
Peel adhesion initial: for cotton, the initial peel is preferred to be in the range of 200-500 g/lineal inch (200-500 g/2.54 cm).
Peel adhesion retention or ageing test: the samples (fabric/adhesive/Mylar) are put in an oven at 40°C/8 hours under a load of 160 g/sq. in (160 g/6.45 cm²). Peel adhesion is determined after 1 hour conditioning at 23°C -50 %RH. Occurrence of adhesive transfer is also reported as none or transfer.
Adhesive transfer: the samples (fabric/adhesive/Mylar) are put in oven at 40 °C/24 hours under a load of 800 g/sq. in. (800 g/6.45 cm²). Peel adhesion is determined after 1 hour conditioning at 23 °C- 50 %RH. Occurrence of adhesive transfer is also reported as none or transfer.

The SAFT (shear adhesion failure temperature) was measured by 1" x 1" (6.45 cm²) Mylar to Mylar lap joint with a 1 kg weight. SAFT measures the temperature at which the lap shear assembly fails under load. Rolling Ball Tack (RBT) is the distance a steel ball rolls on the adhesive film with a standard initial velocity (Pressure Sensitive Tape Council Test No. 6). Small numbers indicate aggressive tack. Holding Power (HP) is the time required to pull a standard area (½ in. x ½ in. (1.61 cm²)) of tape from a standard test surface (steel, Kraft paper) under a standard load (1 kg), in shear at 2° (Pressure Sensitive Tape Council Method No. 7). Long times indicate high adhesive strength. 180° peel was determined by Pressure Sensitive Tape Council Method No. 1. Large numbers indicate high strength when peeling a test tape from a steel substrate. Polyken probe tack (PPT) was determined by ASTM D-2979. Loop tack (LT) was determined using PSTC-5 loop tack method. High numbers for PPT and LT indicate aggressive tack. T-peel is measured by ASTM D-1876.

### Example 1

This series of experiments compares the adhesive properties of several interpolymers formulations. Formulation F-1 has no adhesive transfer after the adhesion initial test but only very slight adhesive transfer for the retention and transfer tests, which is still acceptable. The best results are obtained with formulation F-2 which combines a low hot-melt viscosity with good adhesive properties, namely without adhesive transfer. At higher polymer levels Formulation F-3, namely 20%, the adhesive properties remain good but the viscosity is much higher. Formulation F-5, containing 20 %wt of ENGAGE 8400 shows adhesive transfer during the transfer test. At lower metallocene interpolymer concentrations Formulation F-6, namely 15.8%, the formulation properties are worse, as slight adhesive transfer is already observed for the adhesion initial test, indicating that strong adhesive transfer will appear for the other tests.

**Table 1**

| Formulation | F-1 | F-2 | F-3 | F-5 | F-6 |
|---|---|---|---|---|---|
| EXACT 4049 | 100 | | | | |
| ENGAGE 8200 | | 100 | 100 | | |
| ENGAGE 8400 | | | | 100 | 100 |
| PICCOTAC 95 | 349 | 349 | 275 | 275 | 349 |
| TUFFLO 6056 | 183 | 183 | 125 | 125 | 183 |
| IRGANOX 1010 | 3 | 3 | 3 | 3 | 3 |
| | | | | | |
| Hot Melt Visc. cPs 177 °C | 4,620 | 2,870 | 7,640 | | 662 |
| 180 Peel (steel) | 5.8 | | | | 3.6 |
| 180 Peel Failure | Part Coh | | | | Coh |
| HP Steel, 1kg (min) | 3 | | | | 0.5 |
| HP Failure | Coh | | | | Coh |
| SAFT Mylar 0.5kg | 34 | | | | 28 |
| Loop Tack oz/in (N/m) | 133 (1456) | | | | 134 (1467) |

| Adhesion initial | | | | | |
|---|---|---|---|---|---|
| T-Peel (cotton) pli | 0.8 | 0.8 | 07 | 0.7 | 1.3 ST |
| T-Peel (cotton) N/m | 140 | 140 | 123 | 123 | 228 ST |
| T-Peel (nylon) pli | 0.7 | 0.8 | 0.7 | 0.8 | 1.2 ST |
| T-Peel (nylon) N/m | 123 | 140 | 123 | 123 | 210 ST |

| Retention | | | | | |
|---|---|---|---|---|---|
| Aged T-Peel (cotton) pli | 1.4 ST | 0.8 | 0.7 | 0.8 | |
| Aged T-Peel (cotton) N/m | 245 | 140 | 123 | 140 | |
| Aged T-Peel (nylon) pli | 1.2 ST | 1.2 | 1 | 1.3 | |
| Aged T-Peel (Nylon) N/m | 210 | 210 | 175 | 228 | |

| Transfer | | | | | |
|---|---|---|---|---|---|
| 24hr. T-Peel (cotton) pli | 1.5 ST | 1.4 | 1.1 | 1.9 T | |
| 24hr.T-Peel (cotton) N/m | 263 ST | 245 | 193 | 333 | |
| 24hr. T-Peel (nylon) pli | 1.6 ST | 1.5 | 1.6 | 2.1 T | |
| 24 hr T-Peel (nylon) N/m | 280 ST | 263 | 280 | 368 | |
| ST = slight transfer T= transfer Coh = cohesive | | | | | |

### Example 2

The data in Table 2 show that the formulation F-2 based on ENGAGE 8200 has a very attractive viscosity temperature profile. The viscosity (in cPs)remains low at low temperature even if it is a little bit higher than ENGAGE 8400, F-6. ENGAGE 8200 formulation F-2 has a lower viscosity temperature profile than EXACT 4049, F-1.

**Table 2**

| Temperature °F (°C) | E-8200/15.7% | E-8200/20% | EX-4049/15.7% | EN-8400/15.7% |
|---|---|---|---|---|
| 225 (107) | 36,150 | 110,200 | | |
| 250 (121) | 19,750 | 57,000 | 34,000 | 4,510 |
| 275 (135) | 11,420 | 32,250 | 19,900 | 2,550 |
| 300 (149) | 6,640 | 18,550 | 11,740 | 1,545 |
| 325 (163) | 4,210 | 11,360 | 7,620 | 983 |
| 350 (177) | 2,870 | 7,640 | 4,850 | 659 |

## Claims

1. A hot melt pressure sensitive adhesive which comprises:
(a) from 5 to less than 30 percent by weight basis the total of (a), (b) and (c) of a homogeneous linear or substantially linear interpolymer of ethylene and at least one C₃-C₂₀ alpha olefin having a density of at least 0.85 grams per cubic centimetre and a melt flow index below 30 dg/min; and
(b) from 50 to 80 percent by weight basis the total of (a), (b) and (c) of a tackifying resin; and
(c) from 5 to 35 percent by weight basis the total of (a), (b) and (c) of a plasticizer.

2. The adhesive of claim 1 which comprises from 20 to 35 percent by weight of the plasticizer basis the total of (a), (b) and (c).

3. The adhesive of claims 1 or 2 which comprises from 20 to less than 30 percent by weight of the interpolymer basis the total of (a), (b) and (c).

4. The adhesive of any one of claims 1 to 3, wherein the tackifying resin has an aromaticity such that the MMAP cloud-point is at least 70 °C.

5. The adhesive of claims 1 or 2, which comprises from 10 to 15 percent by weight of the interpolymer basis the total of (a), (b) and (c).

6. The hot melt adhesive of any one of claims 1-5, wherein the viscosity of the adhesive is from 100 to 10,000 cPs at 177 °C and the adhesive exhibits no transfer after being peeled away from a fabric.

7. The adhesive of claim 6 wherein the viscosity of the adhesive is from 600 to 6000 cPs at 177 °C.

8. An absorbent article which incorporates an adhesive according to any one of claims 1-7.

## Patentansprüche

1. Selbstklebender, Heißschmelzklebstoff umfassend :
a) von 5 bis weniger als 30 Gewichts-% bezogen auf die Summe aus (a), (b) und (c) eines homogenen linearen oder im Wesentlichen linearen Copolymerisates aus Ethylen und wenigstens einem C₃-C₂₀ Alphaolefin, das eine Dichte von wenigstens 0,85 Gramm pro Kubikzentimeter aufweist und einen Schmelzflussindex von weniger als 30 dg/min ; und
b) von 50 bis 80 Gewichts-% bezogen auf die Summe aus (a), (b) und (c) eines Harzes zur Erhöhung der Klebrigkeit ; und
c) von 5 bis 35 Gewichts-% eines Weichmachers bezogen auf die Summe aus (a), (b) und (c).

2. Klebstoff nach Anspruch 1, der 20 bis 35 Gewichts-% des Weichmachers bezogen auf die Summe aus (a), (b) und (c) umfasst.

3. Klebstoff nach den Ansprüchen 1 oder 2, der von 20 bis weniger als 30 Gewichts-% des Copolymerisates bezogen auf die Summe aus (a), (b) und (c) umfasst.

4. Klebstoff nach einem der Ansprüche 1 bis 3, wobei das Harz zur Erhöhung der Klebrigkeit eine derartige Aromatizität aufweist, dass der MMAP Trübungspunkt wenigstens 70°C beträgt.

5. Klebstoff nach den Ansprüchen 1 oder 2, der von 10 bis 15 Gewichts-% des Copolymerisates bezogen auf die Summe aus (a), (b) und (c) umfasst.

6. Heißschmelzklebstoff nach einem der Ansprüche 1 bis 5, wobei die Viskosität des Klebstoffs von 100 bis 10000 cPs bei 177°C beträgt und wobei der Klebstoff nachdem er von einem Gewebe abgezogen wurde keine Rückstände hinterlässt.

7. Klebstoff nach Anspruch 6, wobei die Viskosität des Klebstoffs von 600 bis 6000 cPs bei 177°C beträgt.

8. Absorptionsfähiger Gegenstand, der einen Klebstoff nach einem der Ansprüche 1 bis 7 enthält.

## Revendications

1. Adhésif sensible à la pression thermofusible qui comprend :
(a) de 5 à moins de 30% en poids par rapport au total de (a), (b) et (c) d'un interpolymère d'éthylène linéaire ou sensiblement linéaire homogène et d'au moins une alpha-oléfine en C₃-C₂₀ ayant une densité d'au moins 0,85 g par centimètre cube et un indice de fluidité en dessous de 30 dg/min; et
(b) de 50 à 80% en poids par rapport au total de (a), (b) et (c) d'une résine assurant un collage; et
(c) de 5 à 35% en poids par rapport au total de (a), (b) et (c) d'un plastifiant.

2. Adhésif suivant la revendication 1, qui comprend de 20 à 35% en poids du plastifiant par rapport au total de (a), (b) et (c).

3. Adhésif suivant l'une ou l'autre des revendications 1 et 2, qui comprend de 20 à moins de 30% en poids de l'interpolymère par rapport au total de (a), (b) et (c).

4. Adhésif suivant l'une quelconque des revendications 1 à 3, dans lequel la résine assurant un collage a une aromaticité de telle sorte que le point de trouble MMAP soit d'au moins 70°C.

5. Adhésif suivant l'une ou l'autre des revendications 1 et 2, qui comprend de 10 à 15% en poids de l'interpolymère par rapport au total de (a), (b) et (c).

6. Adhésif thermofusible suivant l'une quelconque des revendications 1 à 5, dans lequel la viscosité de l'adhésif est de 100 à 10.000 cPs à 177°C et l'adhésif ne montre pas de transfert après être arraché d'un tissu.

7. Adhésif suivant la revendication 6, dans lequel la viscosité de l'adhésif est de 600 à 6000 cPs à 177°C.

8. Article absorbant qui incorpore un adhésif suivant l'une quelconque des revendications 1 à 7.
